# EUROPÄISCHE PATENTSCHRIFT

(11) **EP 2 959 887 B1**
(45) Veröffentlichungstag und Bekanntmachung des Hinweises auf die Patenterteilung: **14.11.2018**
(21) Anmeldenummer: 14174604.0
(22) Anmeldetag: 26.06.2014
(51) Int. Cl.: A61K 9/16, A61K 9/20, A61K 9/24, A61K 31/495

(54) **Arzneimittel zur Behandlung von Schwindel verschiedener Genese**
Medication for treating dizziness due to various causes
Médicament pour le traitement des vertiges de diverses origines

(43) Veröffentlichungstag der Anmeldung: 30.12.2015
(73) Patentinhaber: Hennig Arzneimittel GmbH&Co. Kg, 65439 Flörsheim am Main (DE)
(72) Erfinder: Przyklenk, Karl-Heinz, 64521 Groß-Gerau (DE); Dr. Grewe, Jan Christoph, 64297 Darmstadt (DE)
(74) Vertreter: Fuchs Patentanwälte Partnerschaft mbB

(56) Entgegenhaltungen:
- WO-A1-2014/001268
- WO-A1-2014/102253
- DE-A1-102005 014 141
- DE-A1-102011 051 308

## Beschreibung

Die vorliegende Erfindung betrifft eine Arzneiform, die geeignet ist, einen Wirkstoff über einen längeren Zeitraum hinweg freizusetzen. Dabei wird eine zweistufige Wirkstofffreisetzung genutzt, um eine allzu häufige Einnahme der Arzneiform entbehrlich zu machen. Die Arzneiformen dieser Erfindung dienen der Behandlung von Schwindel beim Menschen. Sie werden zu oder nach einer Mahlzeit verabreicht. Das erfindungsgemäße Konzept sieht vor, dass die Arzneiform einen formstabilen Anteil aufweist, der sich nach Einnahme der Arzneiform durch den Patienten in den mit Speisebrei gefüllten Magen während mehrerer Stunden nicht auflöst. Dadurch verbleibt dieser formstabile Anteil im Magen bis dieser vermittelt durch die so genannten House Keeper-Kontraktionen entleert wird. Ein anderer Anteil der Arzneiform ist so ausgestaltet, dass er sich binnen kurzer Zeit nach Einnahme durch den Patienten auflöst. So wird eine schnelle Anflutung des Wirkstoffes mit entsprechenden Plasmaspiegeln gewährleistet.

### Stand der Technik

Aus dem Stand der Technik sind Arzneiformen zur Behandlung von Schwindel bekannt, die dazu bestimmt sind einen Wirkstoff über einen längeren Zeitraum freizusetzen. Ein durchschlagender Erfolg für Wirkstoffe, die zur Behandlung von Schwindel geeignet sind, ist bislang allerdings ausgeblieben, zumal diese Wirkstoffe schwer zu formulieren sind.

WO 2012/150246 A1 beschreibt Arzneiformen zur Behandlung von Schwindel, die mehrere wirkstoffhaltige Einheiten umfassen, mit denen eine getaktete Freisetzung erzielt werden soll. Die Einheiten können Schichten einer Schichttablette, Pellets, Mikrotabletten oder dergleichen sein. Die wirkstoffhaltigen Einheiten haben vorzugsweise kleine Durchmesser von wenigen Millimetern. Dadurch soll eine bessere Löslichkeit im Darm erzielt werden, weil die wirkstoffhaltigen Einheiten eine große Oberfläche haben. Die Arzneiform soll eine mehrmals tägliche Gabe des Wirkstoffes simulieren. Zu diesem Zweck umfasst sie einen Anteil, der sich im Magen schnell löst, und einen langsam löslichen Anteil. Allerdings bedarf es für die Funktion dieser Arzneiform eines niedrigen pH-Wertes im Magen (S.19, Abs.1). Dies zeigt sich auch anhand der Beispielarzneiformen, deren Zerfall in 0,1 N HCl ermittelt wurde. Nach einer Mahlzeit ist der pH-Wert im Magen allerdings erhöht. Somit kann diesem Dokument eine Gabe nach einer Mahlzeit nicht entnommen werden. Außerdem sind die Einheiten nicht geeignet, nach der Einnahme im Magen zu verbleiben, weil sie einen kleinen Durchmesser haben. Sie können also den Pylorussphinkter passieren. Die in diesem Dokument des Standes der Technik beschriebenen Arzneiformen sind mithin nicht geeignet, sowohl eine verlängerte Freisetzung des Wirkstoffes als auch eine entsprechende Initialdosis des Wirkstoffes zu gewährleisten. Die Ausgestaltung der verschiedenen Kompartimente in der Arzneiform führt dazu, dass die einzelnen partikulären Einheiten aus dem Magen relativ schnell in den Darm gelangen. Die wirkstoffhaltigen Einheiten werden dann durch den Darm transportiert und verlassen relativ bald die Bereiche des Magen-Darm-Trakt, in denen der Wirkstoff resorbiert werden kann. In unteren Darmabschnitten wird der Wirkstoff in der Regel schlechter resorbiert und schlechter aus der Arzneiform gelöst, weil das Flüssigkeitsangebot stark eingeschränkt ist. Dies ist insbesondere der Fall, wenn die Arzneiform nicht zu oder nach einer Mahlzeit eingenommen wird und wenn die Arzneiform so ausgestaltet ist, dass sie den Magen bereits kurz nach der Einnahme verlässt. Die erfindungsgemäßen Arzneiformen hingegen sind so ausgestaltet, dass wenigstens der formstabile Anteil im Magen des Patienten verbleibt und dort wenigstens so lange gehalten wird, wie eine Mindestmenge an Speisebrei sich im Magen befindet. Die House Keeper-Kontraktionen setzen in der Regel erst ein, wenn ein Großteil des Speisebreis bereits in den Dünndarm befördert wurde. Die erfindungsgemäße Arzneiform macht sich durch ihr Konzept mit einem formstabilen Anteil zu Nutze, dass der Magen einen Anteil des Speisebreis zurückhält und partikuläre Bestandteile mit einem Durchmesser von mehr als 2 mm nicht durch den Pylorussphinkter passieren lässt.

WO 2012/175737 A1 betrifft eine Wirkstoffmatrix und eine Arzneiform, welche die Matrix enthält. Das Vermischen des Wirkstoffes mit dem Speisebrei nach einer Mahlzeit wird thematisiert (S.2, letzter Abs.). Die Matrix soll eine kontinuierliche Freisetzung während der Passage durch den GI-Trakt ermöglichen (S.9, Abs.2). Es wird erwähnt, dass der Wirkstoff mit dem Speisebrei transportiert werden kann (S.17, Abs.2, 5+6). Die Verweildauer im Magen soll erhöht werden. Eine Mantel-Kern-Tablette wird erwähnt (S.18, Abs.4). Es wird eine Arzneiform beschrieben, die zwei Wirkstoffportionen umfasst, eine innerhalb und eine außerhalb der Matrix (S.21, Abs.6). Dabei kann die Matrix einen Überzug haben (S.24, Abs.3). Es soll offenbar eine dreistufige Freisetzung erzielt werden. In diesem Dokument des Standes der Technik wird allerdings geschrieben, dass die betroffene Arzneiform bereits im Magen einen Großteil des Wirkstoffes freisetzen soll. Die Freisetzung soll aus einer Matrix erfolgen, die ein Bestandteil der Arzneiform ist. Es wird als besonders wichtig beschrieben, dass sich die Wirkstoffmatrix in Magen-Darm-Trakt sehr schnell auflöst. Offenbar wird ein formstabiler Anteil nicht empfohlen. Der galenische Ansatz hinter der in diesem Dokument des Standes der Technik beschriebenen Arzneiform ist also gänzlich unterschiedlich von dem hier erfindungsgemäßen. Erfindungsgemäß wird die Arzneiform zu einem bestimmten Anteil im Magen nicht aufgelöst, sondern behält ihre strukturelle Integrität. Der Wirkstoff, der sich in diesem Anteil der Arzneiform befindet, wird somit nicht gelöst und nicht mit dem Speisebrei vermischt. Zwar wird auch eine Ausführungsform beschrieben, in welcher die Arzneiform zwei Wirkstoffportionen umfasst, welche den Wirkstoff an unterschiedlichen Orten freisetzen. Doch bezieht sich dieser Anteil der Offenbarung ganz offensichtlich auf überzogene Pellets oder Tabletten.

WO 2013/030119 A1 beschreibt Darreichungsformen mit stabilisierten Wirkstoffpartikeln. Die Lösungsgeschwindigkeit des Wirkstoffs Cinnarizin soll verbessert werden. Die Darreichungsform ist eine Retardarzneiform. Ein Dosierschema wird nicht empfohlen.

WO 2012/175747 A1 betrifft ein Herstellungsverfahren für eine Arzneiform, die mehrere Untereinheiten umfasst. Darunter können auch Mantel-Kern-Tabletten verstanden werden (S.46, letzter Abs.). Die Einnahme nach einer Mahlzeit wird erwähnt (S.15, Abs.1). Eine mehrmals tägliche Gabe soll entbehrlich sein (S.47, Abs.1). Die in diesem Dokument des Standes der Technik beschriebene Arzneiform soll besonders von Pellets und Mikrotabletten profitieren, die ihrerseits bereits einen so kleinen Durchmesser haben, dass sie vom Magen in den Darm übertreten können. Es wird somit ein gänzlich anderes Konzept verfolgt als das erfindungsgemäße.

WO 2014/001268 A1 betrifft eine Arzneiform zur verlängerten Freisetzung. Die Arzneiform hat mehrere Kompartimente, wie etwa eine Schichttablette. Es wird auf die Probleme im Hinblick auf die Einnahme nach einer Mahlzeit eingegangen (S.1, letzter Abs.). Angeblich sei der Ausgangs-pH-Wert im Magen erst nach bis zu 90 Minuten wieder erreicht. Die Arzneiformen haben eine lange Verweildauer im Magen (S.2, Abs.4). Die Schichttablette kann eine Mantel-Kern-Tablette sein. Es wird auch eine Magenverweilform beschrieben (S.4, Abs.1 bis S.5, Abs.3). Es wird eine nahezu lineare Freisetzung angestrebt. Die in diesem Dokument des Standes der Technik beschriebene Arzneiform unterscheidet sich wesentlich von den erfindungsgemäßen Arzneiformen dadurch, dass eine Verabreichung zu oder nach den Mahlzeiten nicht als vorteilhaft erkannt wurde und dadurch, dass die darin vorgesehenen verschiedenen kompatiblen der Arzneiform (Schichttablette, Mantel-Kern-Tablette) nicht beide einen Anteil des Wirkstoffes aufweisen. Damit ist eine Initialdosierung des Wirkstoffes so wie sie erfindungsgemäß vorgesehen ist, nicht zu verwirklichen.

### Aufgabe

Mit den Arzneiformen aus dem Stand der Technik war es bisher nicht möglich, einen Plasmaspiegel des Wirkstoffes bei Patienten zu erzielen, der eine zufriedenstellende Wirkdauer bei Vermeidung von zu hohen Plasmaspiegeln verwirklicht hat und einen hinreichend frühen Wirkeintritt nach Einnahme sicher stellt.

Daher ist es die Aufgabe der vorliegenden Erfindung, eine Arzneiform bereit zu stellen, die es erlaubt, einen Wirkstoff so zu verabreichen, dass eine mehr als zweimal tägliche Gabe entbehrlich ist, ohne dass zu hohe Plasmaspiegel eintreten, wobei ein früher Eintritt der Wirkung sicher gestellt ist.

### Lösung

Diese Aufgabe wird durch die Gegenstände der Patentansprüche gelöst.

Die erfindungsgemäße Arzneiform umfasst
- einen Wirkstoff oder ein Derivat, Salz und/oder Prodrug davon,
- einen formstabilen Anteil, der sich in einem wässrigen Medium bei einem pH-Wert von <5 während einer Dauer von 2 Stunden auch unter Rühren nicht auflöst und unter diesen Bedingungen nicht in einem Maße erodiert, dass sein kleinster Durchmesser auf einen Wert von kleiner 5 mm, bevorzugt kleiner 4 mm und besonders bevorzugt kleiner 2 mm sinkt,
- einen magensaftlöslichen Anteil, der sich in einem wässrigen Medium bei einem pH-Wert von <5 während einer Dauer von weniger als 2 Stunden auflöst oder unter diesen Bedingungen in einem Maße erodiert, dass sein größter Durchmesser auf einen Wert von <2 mm sinkt.

Das wässrige Medium ist vorzugsweise die Phosphat-Pufferlösung pH 4,5 R des Europäischen Arzneibuchs (Ph.Eur 7.0). Eine geeignete Testapparatur ist die Apparatur für die Bestimmung der Zerfallszeit von Tabletten gemäß Europäischen Arzneibuch (Ph.Eur 7.0, Kapitel 2.9.1). Gerührt wird gegebenenfalls mit einer Blattrührerapparatur gemäß Europäischen Arzneibuch (Ph.Eur. 7.0, Kapitel 2.9.3).

Sowohl der formstabile als auch der magensaftlösliche Anteil enthalten eine Teilmenge des Wirkstoffes. Die Arzneiform ist zur Anwendung in einem Verfahren zur Behandlung von Schwindel beim Menschen durch höchstens zweimal tägliche Verabreichung an einen Patienten zu oder nach einer Mahlzeit bestimmt. Die Verabreichung erfolgt vorzugsweise in einem Zeitfenster von bis zu 1 Stunde nach Einnahme einer Mahlzeit.

Die Arzneiform dieser Erfindung setzt also einen Teil der Wirkstoffmenge bereits kurz nach der Einnahme durch den Patienten aus dem magensaftlöslichen Anteil frei. Dadurch wird eine Initialdosis des Wirkstoffes sofort nach der Einnahme frei. Da die Arzneiform zu oder nach einer Mahlzeit eingenommen werden soll, ist der pH-Wert im Magen erhöht. Schließlich wird die Magensäure durch den Speisebrei verdünnt. Daher ist es erforderlich, dass der magensaftlösliche Anteil der Arzneiform sich bei einem pH-Wert von kleiner 5 bereits auflöst bzw. erodiert. Der Speisebrei wird nach und nach durch den Pylorussphinkter in den Dünndarm abgegeben. Dort gelangt der Wirkstoff zur Resorption und führt zu einem entsprechenden Plasmaspiegel des Wirkstoffes, so dass dessen Wirkung eintreten kann.

Der formstabile Anteil der Arzneiform zeichnet sich dadurch aus, dass er sich unter den angegebenen Bedingungen bei Einnahme zu oder nach einer Mahlzeit nicht sofort auflöst bzw. erodiert. Vielmehr behält der formstabile Anteil eine Größe, die es ihm nicht erlaubt, durch den Pylorussphinkter in den Dünndarm transportiert zu werden. Hierfür ist es erfindungsgemäß vorgesehen, dass der formstabile Anteil auch nach einer Verweildauer von 2 Stunden im Magen bei einem pH-Wert von kleiner 5 nicht so stark aufgelöst oder erodiert wird, dass sein kleinster Durchmesser einen Wert von kleiner 2 mm annimmt. Dadurch wird erreicht, dass der formstabile Anteil im Magen verbleibt bis dieser den Speisebrei im wesentlichen vollständig in den Dünndarm abgegeben hat. Sobald dies der Fall ist befördert der Magen diejenigen Nahrungsbestandteile, die sich noch im Wagen befinden im Wege einer so genannten House Keeper Kontraktion in den Dünndarm.

Der formstabile Anteil gibt nun im Dünndarm weiterhin Wirkstoff ab, so dass dieser zur Resorption gelangt. Dadurch wird erzielt, dass weiterhin ein ausreichend hoher Plasmaspiegel des Wirkstoffes bereitgestellt wird.

Erfindungsgemäß bevorzugt ist der formstabile Anteil der Arzneiform so ausgestaltet, dass der Wirkstoff über einen verlängerten Zeitraum aus dem formstabilen Anteil freigesetzt wird. Zu diesem Zweck umfasst der formstabile Anteil vorzugsweise wenigstens einen Emulgator.

Die Mahlzeit weist hinsichtlich ihrer Zusammensetzung vorzugsweise folgende Eigenschaften auf:

| | |
|---|---|
| Kohlenhydrate: | >50 g |
| Fett: | >10 g |
| Protein: | >10 g |

Der Anteil des Wirkstoffes in dem magensaftlöslichen Anteil entspricht vorzugsweise etwa 20% bis 65% der Wirkstoffmenge in dem formstabilen Anteil. Die erfindungsgemäße Arzneiform ist vorzugsweise frei von Antioxidantien und/oder Komplexbildnern.

### Wirkstoff

Der Wirkstoff in der erfindungsgemäßen Arzneiform kann grundsätzlich beliebig ausgewählt werden. Die erfindungsgemäße Arzneiform ist allerdings darauf zugeschnitten, einen Wirkstoff entsprechend freizusetzen, der sich im pH-Bereich des Dünndarms unter normalen Umständen schlecht löst. Der Wirkstoff weist vorzugsweise in wässriger Lösung bei pH 7 und 22°C eine Löslichkeit von weniger als 0,01 mg/ml auf. Der Wirkstoff hat also vorzugsweise eine geringe Löslichkeit, er ist in Übereinstimmung mit der Löslichkeitsklassifikation des Europäischen Arzneibuchs "praktisch unlöslich" (Ph. Eur. 7, 2011). Bevorzugt beträgt die Löslichkeit bei pH 7 und 22°C in wässriger Lösung höchstens 0,005 mg/ml, weiter bevorzugt höchstens 0,001 mg/ml und noch mehr bevorzugt sogar weniger als 0,001 mg/ml.

Unter Löslichkeit wird die Konzentration des gelösten Stoffes in einer gesättigten Lösung bei einer bestimmten Temperatur verstanden. Die Löslichkeit des Wirkstoffs bezieht sich erfindungsgemäß auf die Löslichkeit in wässriger Lösung bei 22°C unter Normaldruck. Diese Temperaturangabe sowie alle nachfolgenden Temperaturangaben umfassen erfindungsgemäß bevorzugt den konkret angegebenen Wert einschließlich einer Spanne von +/- 3°C um den angegebenen Temperaturwert. Nachfolgende Löslichkeitsangaben beziehen sich, soweit nichts anderes angegeben ist, auf die Löslichkeit unter Normaldruck. Die Bestimmung erfolgt in den Standard- beziehungsweise Pufferlösungen des Europäischen Arzneibuchs (Ph. Eur. 7) mit entsprechendem pH-Wert.

Die wässrige Lösung zur Erzielung eines pH-Wertes von 7 umfasst einen Phosphatpuffer und Wasser. Die Zusammensetzung ist dem Europäischen Arzneibuch zu entnehmen (Ph. Eur. 7; Phosphat-Pufferlösung pH 7.0 R). Die Messung des pH-Wertes erfolgt mit dem Fachmann bekannten Verfahren, beispielsweise mittels eines kommerziell erhältlichen pH-Meters.

Zur Löslichkeitsbestimmung werden dem Fachmann bekannte Verfahren eingesetzt wie die Bodensatzmethode, die Temperaturmethode oder die Verteilungsmethode. Insbesondere geeignet ist die Verteilungsmethode, bei der der Wirkstoff in einem zweiten Lösungsmittel gelöst wird, welches nicht mischbar ist mit der jeweiligen wässrigen Lösung, für die die Löslichkeit des Wirkstoffs ermittelt werden soll. Die beiden Flüssigkeiten werden bis zur Gleichgewichtseinstellung intensiv geschüttelt und dann getrennt.

Der Wirkstoff ist vorzugsweise ausgewählt aus der BCS-Klasse II oder BCS-Klasse IV, besonders bevorzugt ist der Wirkstoff ein Wirkstoff der BCS-Klasse II. Gerade solche Wirkstoffe profitieren besonders von den erfindungsgemäßen Arzneiformen, weil die Auflösung des Wirkstoffs im Dünndarm besonders gefördert wird.

Der Wirkstoff weist vorzugsweise wenigstens eine basische funktionelle Gruppe auf. Gerade solche funktionellen Gruppen, die gewöhnlich mit pH-abhängiger Löslichkeit einhergehen, bereiten bei der Formulierung von Arzneiformen mit verlängerter Freisetzung bislang erhebliche Schwierigkeiten.

Eine Gruppe ist insbesondere basisch, wenn sie Protonen aufnehmen kann (Brönsted-Base). Im Sinne der Erfindung werden nur solche Gruppen als basische funktionelle Gruppen bezeichnet, die einen pK_{B}-Wert von höchstens 9, weiter bevorzugt einen pK_{B}-Wert von höchstens 8,5 und mehr bevorzugt einen pK_{B}-Wert von höchstens 8 haben.

Die basische funktionelle Gruppe ist bevorzugt ausgewählt aus aliphatischen stickstoffhaltigen Gruppen oder einem stickstoffhaltigen Heterozyklus. Unter dem Begriff "stickstoffhaltiger Heterozyklus" werden im Sinne dieser Erfindung stickstoffhaltige Gruppen verstanden, die Bestandteil von aromatischen Kohlenwasserstoffen sind. Die aromatischen Kohlenwasserstoffe können neben der stickstoffhaltigen Gruppe noch weitere Heteroatome als Ringglieder umfassen. Bevorzugte "stickstoffhaltige Heterozyklen" sind aber solche, die neben Stickstoff nur Kohlenstoffatome als Ringglieder enthalten. Aliphatische stickstoffhaltige Gruppen sind vorzugsweise ausgewählt aus Aminogruppen und Guanidingruppen. Der Begriff "Aminogruppe" umfasst primäre, sekundäre und tertiäre Aminogruppen. Auch Stickstoffatome in gesättigten oder teilweise ungesättigten cyclischen Kohlenwasserstoffen, die neben dem Stickstoffatom noch weitere Heteroatome einschließlich weiterer Stickstoffatome als Ringglieder aufweisen können, stellen eine Aminogruppe dar.

Ganz besonders bevorzugt ist die basische funktionelle Gruppe ausgewählt aus Aminogruppe, Guanidingruppe und stickstoffhaltiger Heterozyklus. Noch mehr bevorzugt ist die basische funktionelle Gruppe eine Aminogruppe. Solche Wirkstoffe profitieren aufgrund der ausgeprägten pH-abhängigen Löslichkeit besonders von den erfindungsgemäßen Arzneiformen.

Ganz besonders profitieren solche Wirkstoffe von der erfindungsgemäßen Arzneiform, die mehrwertige Basen sind, also wenigstens zwei basische funktionelle Gruppen aufweisen. Besonders bevorzugt enthält der Wirkstoff daher wenigstens zwei basische funktionelle Gruppen, noch mehr bevorzugt genau zwei basische funktionelle Gruppen. Noch weiter bevorzugt weist der Wirkstoff zwei Aminogruppen auf.

Dadurch ist der Wirkstoff, wenn er mit Magensaft in Kontakt kommt, üblicherweise protoniert und somit in diesem sauren Milieu wasserlöslich. Bei höheren pH-Werten im Darm von 6 und mehr hingegen kann sich der Wirkstoff nur schlecht auflösen. Das trifft besonders auf Wirkstoffe zu, die keine sauren funktionellen Gruppen aufweisen.

Weiter bevorzugt enthält der Wirkstoff also keine sauren funktionellen Gruppen. Saure funktionelle Gruppen sind solche, die im ungeladenen Zustand des Wirkstoffs Protonen abgeben können. Insbesondere weist der Wirkstoff keine sauren Gruppen auf mit einem pK_{A}-Wert unter 5. Saure Gruppen umfassen insbesondere Carboxylgruppen, schwefelhaltige Säuregruppen wie Sulfonsäuregruppen und Schwefelsäureester, phosphorhaltige Säuregruppen, Hydroxylgruppen einschließlich phenolischer Hydroxylgruppen und Amidgruppen. Insbesondere sind keine Carboxylgruppen und/oder Hydroxylgruppen im Wirkstoff enthalten. Die Löslichkeit des Wirkstoffs ist also besonders bevorzugt wesentlich von der basischen funktionellen Gruppe vorzugsweise ausgewählt aus Aminogruppe, Guanidingruppe und stickstoffhaltiger Heterozyklus, ganz besonders bevorzugt der wenigstens einen Aminogruppe, geprägt. Jedenfalls profitieren solche Wirkstoffe besonders vom Design der erfindungsgemäßen Arzneiform.

Der Wirkstoff weist bevorzugt bei pH-Werten < 7 und 22°C höhere Löslichkeiten auf als bei pH 7 und 22°C. Der Wirkstoff weist vorzugsweise bei pH 1 und 22°C in wässriger Lösung eine Löslichkeit von mehr als 0,1 mg/ml, weiter bevorzugt wenigstens 0,5 mg/ml, weiter bevorzugt wenigstens 1 mg/ml, noch weiter bevorzugt mindestens 1,3 mg/ml und besonders bevorzugt mindestens 1,5 mg/ml auf. Der Wirkstoff ist also bei einem pH Wert von 1 bevorzugt wenigstens "sehr schwer löslich", weiter bevorzugt wenigstens "schwer löslich" gemäß der Löslichkeitsklassifikation des Europäischen Arzneibuchs (Ph. Eur. 7). Bevorzugt liegt die Löslichkeit des Wirkstoffs bei pH-Werten von mehr als 7 bei höchstens 0,01 mg/ml, weiter bevorzugt bei höchstens 0,001 mg/ml. Beispielsweise beträgt die Löslichkeit des Wirkstoffs bei pH 9 in wässriger Lösung und 22°C vorzugsweise höchstens 0,01 mg/ml und mehr bevorzugt höchstens 0,001 mg/ml.

Als wässrige Lösung zur Erzielung eines pH-Wertes von 1 wird die Salzsäure-Standardlösung des Europäischen Arzneibuchs (Ph. Eur. 7) verwendet aus Salzsäure und Wasser, das heißt eine 0,1 N (0,1 mol/l) Salzsäurelösung. Zur Ermittlung der Löslichkeit des Wirkstoffs bei pH 9 ist die Phosphat-Pufferlösung pH 9,0 R geeignet.

Der erfindungsgemäß eingesetzte Wirkstoff hat vorzugsweise einen n-Octanol-Wasser-Verteilungskoeffizienten (logP_{OW}) bei 20°C von mehr als 1, vorzugsweise mehr als 2, mehr bevorzugt mehr als 3 und besonders bevorzugt mehr als 4, insbesondere mehr als 5. Dieser Verteilungskoeffizient ist ein Maß für die Lipophilie einer Substanz. Wie zuvor erwähnt, profitieren besonders lipophile Wirkstoffe von der Arzneiform dieser Erfindung. Der Vorteil für solche Wirkstoffe ist auch deswegen besonders hoch, weil üblicherweise die Resorption lipophiler Wirkstoffe sehr gut funktioniert, sobald diese Substanzen gelöst sind. Mit anderen Worten ist die Auflösung dieser Wirkstoffe aus einer Arzneiform der geschwindigkeitsbestimmende Schritt bei der Aufnahme des Wirkstoffes in den Körper.

Trotzdem gibt es auch Wirkstoffe, deren Lipophilie so ausgeprägt ist, dass die hier vorgeschlagene Formulierung nicht ausreicht, um eine ausreichende Bioverfügbarkeit sicher zu stellen. Daher weist der Wirkstoff einen n-Octanol-Wasser-Verteilungskoeffizienten (logP_{OW}) bei 20°C von vorzugsweise höchstens 100, weiter bevorzugt höchstens 50, insbesondere höchstens 30, besonders bevorzugt höchstens 15 und insbesondere höchstens 7 auf.

Um die Löslichkeit des Wirkstoffes nach Applikation weiter zu verbessern, ist es erfindungsgemäß bevorzugt, den ersten Wirkstoff in kleiner Partikelgröße zu verwenden. Daher weist der erste Wirkstoff in der Arzneiform dieser Erfindung vorzugsweise mittlere Partikelgrößen von höchstens 1000 nm, weiter bevorzugt höchstens 600 nm und besonders bevorzugt höchstens 300 nm auf. Die mittlere Partikelgröße wird erfindungsgemäß bevorzugt mit der Analysemethode der dynamischen Lichtstreuung ermittelt. Die kleine Partikelgröße ist insbesondere dann von Bedeutung, wenn sich der Wirkstoff im Emulgator nicht oder kaum löst. Dies trifft meist zu, wenn der Emulgator einen besonders großen hydrophilen Anteil hat, wie etwa viele Poloxamere, und/oder der Wirkstoff besonders lipophil ist.

Es ist erfindungsgemäß bevorzugt, dass sich der Wirkstoff im Emulgator nahezu vollständig, besonders bevorzugt vollständig, löst. Dadurch wird eine verbesserte Freisetzung erreicht. Erzielt werden kann dieser Effekt dadurch, dass der lipophile Anteil des Emulgators an die Lipophilie des Wirkstoffes angepasst wird. Wie das erreicht werden kann, ergibt sich aus den weiter unten folgenden Ausführungen zum Emulgator und dessen Molekülstruktur. Der Wirkstoff liegt also besonders bevorzugt vollständig gelöst in der erfindungsgemäßen Arzneiform vor.

Insbesondere Wirkstoffe, die bei Verwendung sofortfreisetzender Arzneiformen wenigstens dreimal täglich verabreicht werden müssen, profitieren von der erfindungsgemäßen Arzneiform. Der Wirkstoff ist also vorzugsweise ein solcher, der bei Einnahme sofortfreisetzender Arzneiformen mindestens 3-mal täglich verabreicht werden muss.

Besonders bevorzugt ist der Wirkstoff ausgewählt aus Wirkstoffen der Wirkstoffklassen Antihistaminika, Antiemetika, Antivertiginosa und/oder Calciumkanalblocker. In einer besonders bevorzugten Ausführungsform ist der Wirkstoff Cinnarizin, ein Cinnarizinsalz und/oder ein Cinnarizinderivat, insbesondere Prodrug. Ganz besonders bevorzugt ist der erste Wirkstoff Cinnarizin.

Der Anteil des Wirkstoffs an der Arzneiform beträgt vorzugsweise wenigstens 1 Gew.-%, weiter bevorzugt wenigstens 2 Gew.-%, mehr bevorzugt wenigstens 3 Gew.-% und besonders bevorzugt wenigstens 5 Gew.-% bezogen auf die Gesamtmasse der erfindungsgemäßen Arzneiform. Ein gewisser Wirkstoffanteil ist notwendig, um die gewünschte pharmakologische Wirkung zu erzielen. Der Wirkstoffanteil darf nicht zu hoch sein, weil die Verbesserung der Wirkstoffauflösung nicht für beliebig hohe Wirkstoffanteile mit hinreichender Sicherheit erzielt werden kann. Daher ist der Wirkstoffgehalt des Wirkstoffs an der Arzneiform in bevorzugten Ausführungsformen auf höchstens 25 Gew.-%, weiter bevorzugt höchstens 18 Gew.-%, mehr bevorzugt höchstens 15 Gew.-% und besonders bevorzugt höchstens 12 Gew.-% bezogen auf die Gesamtmasse der erfindungsgemäßen Arzneiform beschränkt.

Der Wirkstoff wird vorzugsweise in einer Menge von wenigstens 20 mg oder besonders bevorzugt wenigstens 30 mg in der erfindungsgemäßen Arzneiform eingesetzt. Die Menge an erstem Wirkstoff soll vorzugsweise einen Wert von 100 mg und besonders bevorzugt 80 mg in der erfindungsgemäßen Arzneiform nicht übersteigen.

Die Teilmenge des Wirkstoffes in dem magensaftlöslichen Anteil der Arzneiform ist vorzugsweise kleiner als die Teilmenge des Wirkstoffes in dem formstabilen Anteil. Die Teilmenge des Wirkstoffes in dem formstabilen Anteil beträgt vorzugsweise zwischen 55 und 75% der gesamten Wirkstoffmenge in der Arzneiform, besonders bevorzugt zwischen 60 und 70%. Die Restmenge befindet sich dann vorzugsweise in dem magensaftlöslichen Anteil.

### Emulgator

Der Emulgator übernimmt in dem formstabilen Anteil eine Doppelfunktion. Diese Doppelfunktion besteht zum einen darin, die Freisetzung des Wirkstoffes über einen längeren Zeitraum verteilt zu ermöglichen, und zum anderen darin, die Löslichkeit des Wirkstoffes zu unterstützen, mit anderen Worten: die Freisetzung des Wirkstoffes zu steuern. Intensive Untersuchungen haben ergeben, dass diese Doppelfunktion im Rahmen der erfindungsgemäßen Arzneiform besonders gut von dem nachfolgend beschriebenen Emulgator erfüllt wird. Der magensaftlösliche Anteil der Arzneiform enthält vorzugsweise keinen Emulgator, weil ihre Funktion darin besteht, den Wirkstoff möglichst schnell, im Sinne einer Initialdosis freizusetzen.

Damit die Verbesserung der Auflösung und Modifikation der Freisetzung des Wirkstoffanteils besonders gut ausgeprägt ist, soll der Emulgator vorzugsweise einen HLB-Wert von wenigstens 1 und höchstens 16, insbesondere wenigstens 9 und höchstens 14 aufweisen. Der Emulgator ist vorzugsweise nichtionisch.

Es hat sich gezeigt, dass solche Emulgatoren besonders vorteilhafte Ergebnisse erzielen, die ein bestimmtes Strukturmotiv aufweisen. Dieses Strukturmotiv ist eine Polyethylenglykolkette. Es wurde gefunden, dass Emulgatoren mit diesem Strukturmotiv, also wenigstens einer Polyethylenglykolkette, die erfindungsgemäß erwünschte Doppelfunktion erfüllen. Einerseits führen sie bei hohen pH-Werten ab etwa pH 4 zu einer Verbesserung der Löslichkeit des ersten Wirkstoffes. Andererseits führen sie besonders bei niedrigen pH-Werten, nämlich dann, wenn der Wirkstoff vorzugsweise eine höhere Löslichkeit aufweist, zu einer Verzögerung der Freisetzung. Dies ist ein erfindungsgemäß erwünschtes Verhalten, soll doch die Freisetzung des Wirkstoffes aus dem formstabilen Anteil möglichst kontinuierlich über einen langen Zeitraum erfolgen.

Die Polyethylenglykolkette stellt den hydrophilen Teil des Emulgators dar und ist mit einem lipophilen Teil verbunden. Der lipophile Teil kann vorteilhafterweise ein Glyceridrest oder eine andere Polyalkylenoxidkette sein. Die Polyethylenglykolkette hat ferner den Vorteil, dass sie chemisch leicht zu modifizieren, also zu verlängern oder zu verkürzen, ist. Damit kann der hydrophile Anteil an den Wirkstoff angepasst werden.

Als Glyceridreste kommen Mono- und Diglyceridreste in Frage, wobei Diglyceridreste bevorzugt sind. Monoglyceridreste sind unter Umständen, je nach Kettenlänge der beteiligten Fettsäurereste, nicht lipophil genug.

Als Polyalkylenoxidketten kommen insbesondere Polyalkylenoxide in Frage, die ununterbrochene Kohlenstoffketten von wenigstens 3 Kohlenstoffatomen aufweisen. Ein bevorzugtes Beispiel für eine Polyalkylenoxidkette im Emulgator dieser Erfindung ist Polypropylenoxid.

Diese Emulgatoren können mit dem Fachmann bekannten Verfahren erhalten werden. Polyethylenglykolglyceride können beispielsweise durch Reaktion von den entsprechenden Glyceriden mit Polyethylenglykol hergestellt werden. Kommerziell erhältliche Polyethylenglykolglyceride sind beispielsweise Gelucire® 43/01 und Gelucire® 50/13.

Mit anderen Worten: die Emulgatoren, die in der erfindungsgemäßen Arzneiform eingesetzt werden, weisen folgendes Strukturelement auf:

R¹-O-[CH₂-CH₂-O]ₙ-R² (Formel I)

Darin sind R¹ und R² gleich oder verschieden. R¹ und R² sind unabhängig voneinander Wasserstoff, Alkyl, Glycerid oder Polyalkylenoxid. Alkyl ist bevorzugt kurzkettig, d.h. es weist eine Kettenlänge von höchstens 6 Kohlenstoffatomen auf. Bevorzugt sind R¹ und R² unabhängig voneinander Wasserstoff, Glycerid oder Polyalkylenoxid.

Wenn ein Rest R¹ oder R² Wasserstoff ist, sind die Reste R¹ und R² verschieden, d.h. der jeweils andere Rest ist nicht Wasserstoff; und wenn R¹ oder R² Alkyl ist, sind die Reste R¹ und R² verschieden, d.h. der jeweils andere Rest ist nicht Alkyl. Andernfalls hätte das Molekül nicht die erforderliche emulgierende Wirkung. Vorzugsweise sind R¹ und R² weder Wasserstoff noch Alkyl, wenn der jeweils andere Rest Wasserstoff oder Alkyl ist.

n ist die Anzahl der Kettenglieder in der Polyethylenoxidkette. n ist eine ganze Zahl von wenigstens 4, bevorzugt wenigstens 10 und besonders bevorzugt wenigstens 20. Ist n kleiner, so ist der erfindungsgemäß erwünschte Effekt nicht sehr stark ausgeprägt, weil der lipophile Teil des Emulgators zu klein ist. In bevorzugten Ausführungsformen ist n nicht größer als 100, insbesondere nicht größer als 50, weiter bevorzugt nicht größer als 40 oder nicht größer als 30. Es hat sich gezeigt, dass größere Kettenlängen dazu führen, dass der Emulgator vom Pankreatin und Lipasen langsamer abgebaut wird. Dadurch lässt sich die verlängerte Freisetzung steuern. Ist die Kette aber zu lang, setzt die Arzneiform den Wirkstoff nicht schnell genug frei.

Wenn R¹ und/oder R² ein Polyalkylenoxidrest sind, so hat der Rest folgende allgemeine Formel:

-O-[Y-O]ₘ-R³ (Formel II)

Darin ist R³ Wasserstoff oder Alkyl, insbesondere kurzkettiges Alkyl (bis C₆). Y ist eine Alkylengruppe mit einer Kohlenstoffkettenlänge von wenigstens C₃ und vorzugsweise höchstens C₆, weiter bevorzugt höchstens C₄.

m bezeichnet die Anzahl der Kettenglieder in der Polyalkylenoxidkette. m ist vorzugsweise eine ganze Zahl von wenigstens 3, insbesondere wenigstens 5 und besonders bevorzugt wenigstens 10. m soll vorzugsweise eine ganze Zahl von 50, insbesondere 40 und besonders bevorzugt 30 oder 20 nicht überschreiten. In bevorzugten Ausführungsformen sind R¹ und R² Polyalkylenoxidreste.

Wenn R¹ und/oder R² Glyceridreste sind, so hat der Glyceridrest folgende allgemeine Formel:

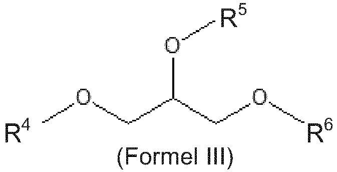

Dabei ist die Formel III an einer der Stellen R⁴, R⁵ oder R⁶ mit dem Strukturelement der Formel I verknüpft. Es ist erfindungsgemäß unerheblich, an welcher der Stellen die Struktur der Formel III mit der Struktur der Formel I verknüpft ist. Es ist also einer der Reste R⁴, R⁵ oder R⁶ die Struktur der Formel I. Für die übrigen beiden Reste gilt dann folgendes:
R⁴ ist vorzugsweise Wasserstoff oder ein Fettsäurerest. Der Fettsäurerest R⁴ weist eine Kettenlänge von vorzugsweise wenigstens C₆, weiter bevorzugt wenigstens C₈ und besonders bevorzugt wenigstens C₁₀ auf. Eine Mindestkettenlänge sollte eingehalten werden damit der Glyceridrest hinreichend lipophil ist. Die Anzahl der Kohlenstoffatome in den Fettsäuren ist vorzugsweise gradzahlig. Die Kettenlänge soll vorzugsweise einen Wert von C₂₂, weiter bevorzugt C₁₈ und besonders bevorzugt C₁₆ nicht überschreiten, weil andernfalls die Löslichkeit des Emulgators beeinträchtigt werden könnte.

R⁵ ist vorzugsweise Wasserstoff oder ein Fettsäurerest. Der Fettsäurerest R⁵ weist eine Kettenlänge von vorzugsweise wenigstens C₆, weiter bevorzugt wenigstens C₈ und besonders bevorzugt wenigstens C₁₀ auf. Eine Mindestkettenlänge sollte eingehalten werden damit der Glyceridrest hinreichend lipophil ist. Die Anzahl der Kohlenstoffatome in den Fettsäuren ist vorzugsweise gradzahlig. Die Kettenlänge soll vorzugsweise einen Wert von C₂₂, weiter bevorzugt C₁₈ und besonders bevorzugt C₁₆ nicht überschreiten, weil andernfalls die Löslichkeit des Emulgators beeinträchtigt werden könnte.

R⁶ ist vorzugsweise Wasserstoff oder ein Fettsäurerest. Der Fettsäurerest R⁶ weist eine Kettenlänge von vorzugsweise wenigstens C₆, weiter bevorzugt wenigstens C₈ und besonders bevorzugt wenigstens C₁₀ auf. Eine Mindestkettenlänge sollte eingehalten werden damit der Glyceridrest hinreichend lipophil ist. Die Anzahl der Kohlenstoffatome in den Fettsäuren ist vorzugsweise gradzahlig. Die Kettenlänge soll vorzugsweise einen Wert von C₂₂, weiter bevorzugt C₁₈ und besonders bevorzugt C₁₆ nicht überschreiten, weil andernfalls die Löslichkeit des Emulgators beeinträchtigt werden könnte.

Vorzugsweise sind zwei der Reste R⁴, R⁵ und R⁶ Fettsäurereste. Entsprechend handelt es sich dann um eine Diglyceridgruppe.

Es ist zu beachten, dass die richtige Kettenlänge der Fettsäurereste stark mit der Anzahl der Kettenglieder in der Polyethylenoxidkette (n) korrespondiert. Ist die Polyethylenoxidkette länger, können auch die Fettsäureketten länger sein.

Die hier beschriebenen Emulgatoren lassen sich durch Reaktion von entsprechenden Mono-, Di- und/oder Triglyceriden mit entsprechenden Polyalkylenoxiden herstellen. Diese Edukte sind kommerziell erhältlich. Die erfindungsgemäßen Emulgatoren sind vorzugsweise Mischungen der genannten Substanzen.

In bevorzugten Ausführungsformen ist der Emulgator ausgewählt aus Gelucire® 50/13, Gelucire® 43/01, Poloxamer 407 und Mischungen daraus, ganz besonders bevorzugt ist Gelucire® 50/13.

Eine gute Lösungsvermittlung wird erzielt, wenn das Verhältnis der Massen von Wirkstoff zu Emulgator in dem formstabilen Anteil wenigstens 1 zu 30, weiter bevorzugt wenigstens 1 zu 20 und besonders bevorzugt wenigstens 1 zu 10 und weiter bevorzugt wenigstens 1 zu 6 beträgt. Das genannte Verhältnis beträgt vorzugsweise höchstens 1 zu 1, weiter bevorzugt höchstens 1 zu 2 und besonders bevorzugt höchstens 1 zu 3. Grundsätzlich gilt, dass eine größere Menge an Emulgator eine bessere Lösungsvermittlung ermöglicht. Es muss aber berücksichtigt werden, dass eine Arzneiform bestimmte Höchstvolumina nicht überschreiten sollte, damit die Einnahme nicht unnötig unangenehm ist. Außerdem kann eine zu große Menge an Emulgator auch dazu führen, dass die Auflösung und/oder die Freisetzung des Wirkstoffes behindert werden.

Der formstabile Anteil der Arzneiform enthält den Emulgator vorzugsweise in einem Anteil von wenigstens 15 Gew.-%, weiter bevorzugt wenigstens 20 Gew.-%, noch weiter bevorzugt wenigstens 22 Gew.-% und besonders bevorzugt wenigstens 25 Gew.-% bezogen auf die Gesamtmasse des formstabilen Anteils. Der Anteil des Emulgators soll vorzugsweise einen Wert von 50 Gew.-%, weiter bevorzugt 40 Gew.-% und besonders bevorzugt 35 Gew.-% bezogen auf die Gesamtmasse des formstabilen Anteils nicht überschreiten. Beim Versuch, das optimale Verhältnis von Lösungsvermittlung und Volumen der Arzneiform zu ermitteln, haben sich diese Mengen als vorteilhaft erwiesen. Der Masseanteil des Emulgators an der Arzneiform beträgt vorzugsweise wenigstens 4 Gew.-%, weiter bevorzugt wenigstens 10 Gew.-% und besonders bevorzugt wenigstens 15 Gew.-%, allerdings bevorzugt höchstens 40 Gew.-%, weiter bevorzugt höchstens 30 Gew.-% und besonders bevorzugt höchstens 25 Gew.-%.

In besonders bevorzugten Ausführungsformen wird anstelle der oben genannten Emulgatoren oder zusätzlich zu den oben genannten Emulgatoren mindestens ein Phospholipid eingesetzt. Das Phospholipid ist vorzugsweise ausgewählt aus Phosphatidylcholinen (Lecithine), Phosphatidylethanolaminen (Kephaline), Phosphatidylserinen, Sphingomyelinen und Mischungen davon. Besonders bevorzugt sind Phosphatidylcholine (Lecithine). Es wurde überraschend gefunden, das Phospholipide ebenfalls die oben beschriebenen vorteilhaften Wirkungen hervorrufen, wie die oben genannten Emulgatoren und synergistisch mit diesen wirken.

Der Emulgator kann also ein Phospholipid sein. Vorzugweise umfasst der Emulgator das Phospholipid zusätzlich zu mindestens einer der oben genannten Emulgatoren. Das Phospholipid verstärkt dabei die vorteilhaften Wirkungen der oben genannten Substanzen.

### Pharmakokinetik und Dosierung

Wie eingangs beschrieben, soll mit der erfindungsgemäßen Arzneiform eine vorteilhafte Pharmakokinetik erzielt werden, die sich durch einen schnellen Wirkeintritt und eine lange Wirkdauer auszeichnet. Bevorzugt wird bei der Anwendung der erfindungsgemäßen Arzneiform eine Pharmakologie tätig erzielt, die durch eine Cₘₐₓ spätestens 8 Stunden nach Einnahme gekennzeichnet ist. Es liegt auf der Hand, dass bei der erfindungsgemäß vorgesehenen Behandlung von Schwindel beim Menschen eine möglichst dauerhafte Wirkung erwünscht ist, die in erster Linie tagsüber von besonderer Bedeutung ist. Somit ist es besonders bevorzugt, dass eine Einnahme der Arzneiform zu oder nach dem Frühstück vorgesehen ist.

Die Arzneiform dieser Erfindung ist geeignet, über einen Zeitraum von mindestens 16 Stunden, weiter bevorzugt mindestens 18 Stunden und besonders bevorzugt mindestens 20 Stunden einen Wirkstoffspiegel bereitzustellen, der einer wirksamen Konzentration des Wirkstoffes entspricht.

Die Verabreichung der erfindungsgemäßen Arzneiform erfolgte weniger als dreimal täglich, vorzugsweise zweimal täglich und besonders bevorzugt nur einmal täglich. Das Patientenkollektiv, bei dem die Arzneiform zum Einsatz kommen soll ist bevorzugt ausgewählt aus Patienten mit einem Lebensalter von mehr als 40 Jahren, weiter bevorzugt mehr als 50 Jahren und besonders bevorzugt mehr als 60 Jahren. In dieser Altersgruppe ist die Behandlung von Schwindel von besonderer Bedeutung.

Arzneimittel nach einem der vorhergehenden Ansprüche, kann als Schichttablette ausgestaltet sein, wobei der formstabile und der magensaftlösliche Anteil je eine Schicht darstellen, oder als Mantel-Kern-Tablette, wobei der formstabile Anteil den Kern und der magensaftlösliche Anteil den Mantel darstellt, dabei kann zwischen Kern und Mantel ein magensaftresistenter Film angeordnet sein.

### Weitere Hilfsstoffe

Um die erfindungsgemäß erwünschten Eigenschaften der Arzneiform sicherzustellen, werden Hilfsstoffe eingesetzt. Ein Hilfsstoff ist der oben bereits diskutierte Emulgatoren. Neben dem Emulator umfasst der formstabile Anteil der Arzneiform vorzugsweise wenigstens ein Strukturmittel und/oder wenigstens ein Sprengmittel. Auch der magensaftlösliche Anteil der Arzneiform enthält vorzugsweise ein Strukturmittel und/oder ein Sprengmittel. In besonders bevorzugten Ausführungsformen ist der Massenanteil an Sprengmittel in dem formstabilen Anteil kleiner als in dem magensaftlöslichen Anteil. Das Sprengmittel fördert das Auseinanderfallen der Arzneiform und trägt so zur Wirkstofffreisetzung bei. In bevorzugten Ausführungsformen enthalten der formstabile und der magensaftlösliche Anteil unterschiedliche Strukturmittel und/oder unterschiedliche Sprengmittel.

### Strukturmittel im formstabilen Anteil

Der Gehalt des Strukturmittels beträgt erfindungsgemäß bevorzugt wenigstens 15 Gew.-%, weiter bevorzugt wenigstens 20 Gew.-% und besonders bevorzugt wenigstens 25 Gew.-% an der Gesamtmasse des formstabilen Anteils. Der Anteil des Strukturmittels soll allerdings vorzugsweise einen Gehalt von 50 Gew.-% weiter bevorzugt 40 Gew.-% und besonders bevorzugt 30 Gew.-% an dem formstabilen Anteil nicht überschreiten.

Geeignete Strukturmittel können anorganischer oder organischer Art sein. Das Strukturmittel ist vorzugsweise ausgewählt aus wenigstens einem natürlichen Polymer, wenigstens einem synthetischen Polymer und Mischungen daraus.

Besonders bevorzugt ist das Strukturmittel in Wasser quellfähig, also in der Lage, durch Wasseraufnahme an Volumen zu gewinnen und somit eine Diffusionsbarriere für den Wirkstoff darzustellen. Bevorzugte Strukturmittel haben ein Quellvermögen von mehr als 1,5, bevorzugt mehr als 3, weiter bevorzugt mehr als 5. Es ist vorteilhaft, wenn das Strukturmittel ein Quellvermögen von höchstens 20 aufweist, bevorzugt höchstens 15. Dies bedeutet, dass das Volumen des gequollenen Strukturmittels vorzugsweise nicht mehr als das 20-fache, bevorzugt nicht mehr als das 15-fache des Volumens des nicht gequollenen Strukturmittels beträgt. Ein zu hohes Quellvermögen des Strukturmittels kann, da dieses vorzugsweise in einem verhältnismäßig hohen Anteil an der Arzneiform enthalten ist, die Freisetzung des Wirkstoffs behindern. Das Quellvermögen kann mit dem Fachmann bekannten Verfahren ermittelt werden, beispielsweise durch mikroskopische Verfolgung der Volumenzunahme bei der Quellung unter jeweils geeigneten Bedingungen zur Initiierung einer Quellung.

Bevorzugt wird ein Strukturmittel verwendet, dass in einer wässrigen Lösung in einem Anteil von 2 Gew.-% bei einer Temperatur von 20°C und einem Druck von 101,325 kPa eine Viskosität von wenigstens 500 mPas aufweist. Die Viskosität wird mit einem Kapillarviskosimeter (DIN 53015) gemessen. Je nach gewünschter Verlängerung der Freisetzung des Wirkstoffes kann es erwünscht sein, auch höhere Viskositäten zu wählen. Je höher die Viskosität des Strukturmittels, desto stärker wird die Modifikation der Freisetzung ausfallen. In besonders bevorzugten Ausführungsformen beträgt die Viskosität des Strukturmittels unter den genannten Bedingungen sogar wenigstens 2500 mPas und besonders bevorzugt wenigstens 3500 mPas. Ist die Viskosität allerdings zu hoch, wird der Wirkstoff unter Umständen zu langsam freigesetzt. Dies könnte dazu führen, dass der Wirkstoff während seiner Passage durch den Magen-Darm-Trakt unter Umständen nicht vollständig freigesetzt wird oder erst in unteren Darmabschnitten, so dass therapeutische Plasmaspiegel nicht erzielt werden könnten. Die Viskosität ist unter den genannten Bedingungen vorzugsweise auf höchstens 200 000 mPas, weiter bevorzugt auf höchstens 120 000 mPas, noch weiter bevorzugt auf höchstens 90 000 mPas und am meisten bevorzugt auf höchstens 15 000 mPas beschränkt.

Bevorzugte Strukturmittel weisen ein mittleres Molekulargewicht (Zahlenmittel M_{N}) von wenigstens 5.000, weiter bevorzugt wenigstens 12.000 und besonders bevorzugt wenigstens 20.000 auf. Strukturmittel mit zu kleinen mittleren Molekulargewichten weisen oft nicht die nötigen Festigkeiten auf, um stabile und abriebfeste Arzneiformen herstellen zu können. Strukturmittel mit zu hohen mittleren Molekulargewichten hingegen lösen sich oft schlechter auf, so dass die Freisetzung des Wirkstoffs behindert werden kann und die Gefahr besteht, dass dieser erst in tiefen Darmabschnitten freigesetzt wird und somit nicht mehr ausreichend resorbiert werden kann. Das Strukturmittel hat vorzugsweise ein mittleres Molekulargewicht von höchstens 250.000, weiter bevorzugt höchstens 200.000 und am meisten bevorzugt höchstens 150.000. Ein denkbares Strukturmittel ist Methocel E4M mit einem Molekulargewicht von ca. 100.000.

Das Strukturmittel ist vorzugsweise ausgewählt aus natürlichen oder synthetischen Polysacchariden, Polymethacrylaten und deren Copolymeren, Polyacrylaten und Mischungen daraus.

Besonders bevorzugt umfasst das Strukturmittel ein natürliches oder synthetisches Polysaccharid. Besonders bevorzugt besteht das Strukturmittel aus einem natürlichen oder synthetischen Polysaccharid. Bevorzugte Polysaccharide weisen mehr als 10 Monosaccharidbausteine auf. Als geeignete Monosaccharidbausteine haben sich Pentosen und Hexosen erwiesen, weiter bevorzugt ausgewählt aus Glucose, Galactose, Xylose, Fructose, Arabinose, Mannose, Mannuronsäure, Guluronsäure, Gulose und Mischungen daraus. Besonders bevorzugte natürliche oder synthetische Polysaccharide sind ausgewählt aus Cellulosen, Cellulosederivaten, Alginaten und Mischungen daraus.

Ganz besonders bevorzugt umfasst das Strukturmittel ein Cellulosederivat. Geeignete Cellulosederivate weisen durchschnittliche Substitutionsgrade von wenigstens 1 auf, weiter bevorzugt wenigstens 1,2 und am meisten bevorzugt wenigstens 1,3. Der Substitutionsgrad bezeichnet die mittlere Anzahl von Substituenten pro Glucoseeinheit der Cellulose. Bei zu geringem Substitutionsgrad können die Quellfähigkeit des Retardierungsmittels und die Viskosität vermindert sein, so dass die Freisetzung des Wirkstoffs unter Umständen nicht mehr ausreichend modifiziert wird.

Bevorzugte Cellulosederivate sind ausgewählt aus Methylcellulose, Hydroxypropylcellulose, Hydroxypropylmethylcellulose und Mischungen daraus. Besonders bevorzugte Cellulosederivate sind ausgewählt aus Methylcellulose, Hydroxypropylmethylcellulose und Mischungen daraus. Ein am meisten bevorzugtes Cellulosederivat ist Methylcellulose. Ganz besonders bevorzugt besteht das Strukturmittel in den formstabilen Anteil aus Methylcellulose.

Besonders bevorzugt umfasst das Strukturmittel keine Polymere mit permanenter Ladung. Besonders bevorzugt weist das Strukturmittel im Wesentlichen keine ionischen Substanzen auf, insbesondere keine Natriumcarboxymethylcellulosen. Dies bedeutet erfindungsgemäß, dass das Strukturmittel weniger als 15 Gew.-%, bevorzugt weniger als 5 Gew.-% und weiter bevorzugt höchstens 1 Gew.-% Substanzen mit permanenter Ladung umfasst. Ganz besonders bevorzugt sind keine Substanzen mit permanenter Ladung im Strukturmittel enthalten. Sind nämlich Substanzen mit permanenten Ladungen im Strukturmittel in zu hoher Menge enthalten, so besteht die Gefahr, dass es zu Prezipitatbildung mit anderen Substanzen mit permanenter Ladung kommt, wie etwa einem geladenen Wirkstoff. Eine Prezipitatbildung kann für die Stabilität der Arzneiform und/oder die Freisetzung des Wirkstoffs nachteilig sein. Besonders bevorzugt enthält also das Strukturmittel und weiter bevorzugt die erfindungsgemäße Arzneiform keine Natriumcarboxymethylcellulose.

### Strukturmittel im magensaftlöslichen Anteil

Der Gehalt des Strukturmittels im magensaftlöslichen Anteil der Arzneiform beträgt erfindungsgemäß bevorzugt wenigstens 20 Gew.-%, weiter bevorzugt wenigstens 25 Gew.-% und besonders bevorzugt wenigstens 30 Gew.-% an der Gesamtmasse des Anteils. Der Anteil des Strukturmittels soll allerdings vorzugsweise einen Gehalt von 55 Gew.-% weiter bevorzugt 50 Gew.-% und besonders bevorzugt 40 Gew.-% an dem magensaftlöslichen Anteil nicht überschreiten.

Geeignete Strukturmittel können anorganischer oder organischer Art sein. Das Strukturmittel ist vorzugsweise ausgewählt aus wenigstens einem natürlichen Polymer, wenigstens einem synthetischen Polymer und Mischungen daraus.

Besonders bevorzugt ist das Strukturmittel in Wasser quellfähig, also in der Lage, durch Wasseraufnahme an Volumen zu gewinnen und somit eine Diffusionsbarriere für den Wirkstoff darzustellen. Bevorzugte Strukturmittel haben ein Quellvermögen von mehr als 1,5, bevorzugt mehr als 3, weiter bevorzugt mehr als 5. Es ist vorteilhaft, wenn das Strukturmittel ein Quellvermögen von höchstens 20 aufweist, bevorzugt höchstens 15. Dies bedeutet, dass das Volumen des gequollenen Strukturmittels vorzugsweise nicht mehr als das 20-fache, bevorzugt nicht mehr als das 15-fache des Volumens des nicht gequollenen Strukturmittels beträgt. Ein zu hohes Quellvermögen des Strukturmittels kann, da dieses vorzugsweise in einem verhältnismäßig hohen Anteil an der Arzneiform enthalten ist, die Freisetzung des Wirkstoffs behindern. Das Quellvermögen kann mit dem Fachmann bekannten Verfahren ermittelt werden, beispielsweise durch mikroskopische Verfolgung der Volumenzunahme bei der Quellung unter jeweils geeigneten Bedingungen zur Initiierung einer Quellung.

Bevorzugt wird ein Strukturmittel verwendet, dass in einer wässrigen Lösung in einem Anteil von 2 Gew.-% bei einer Temperatur von 20°C und einem Druck von 101,325 kPa eine Viskosität von wenigstens 500 mPas aufweist. Die Viskosität wird mit einem Kapillarviskosimeter (DIN 53015) gemessen. Je nach gewünschter Verlängerung der Freisetzung des zweiten Wirkstoffes kann es erwünscht sein, auch höhere Viskositäten zu wählen. Je höher die Viskosität des Strukturmittels, desto stärker wird die Modifikation der Freisetzung ausfallen. In besonders bevorzugten Ausführungsformen beträgt die Viskosität des Strukturmittels unter den genannten Bedingungen sogar wenigstens 2500 mPas und besonders bevorzugt wenigstens 3500 mPas. Ist die Viskosität allerdings zu hoch, wird der Wirkstoff unter Umständen zu langsam freigesetzt. Dies könnte dazu führen, dass der Wirkstoff während seiner Passage durch den Magen-Darm-Trakt unter Umständen nicht vollständig freigesetzt wird oder erst in unteren Darmabschnitten, so dass therapeutische Plasmaspiegel nicht erzielt werden könnten. Die Viskosität ist unter den genannten Bedingungen vorzugsweise auf höchstens 200 000 mPas, weiter bevorzugt auf höchstens 120 000 mPas, noch weiter bevorzugt auf höchstens 90 000 mPas und am meisten bevorzugt auf höchstens 15 000 mPas beschränkt.

Bevorzugte Strukturmittel weisen ein mittleres Molekulargewicht (Zahlenmittel M_{N}) von wenigstens 5.000, weiter bevorzugt wenigstens 12.000 und besonders bevorzugt wenigstens 20.000 auf. Strukturmittel mit zu kleinen mittleren Molekulargewichten weisen oft nicht die nötigen Festigkeiten auf, um stabile und abriebfeste Arzneiformen herstellen zu können. Strukturmittel mit zu hohen mittleren Molekulargewichten hingegen lösen sich oft schlechter auf, so dass die Freisetzung des Wirkstoffs behindert werden kann und die Gefahr besteht, dass dieser erst in tiefen Darmabschnitten freigesetzt wird und somit nicht mehr ausreichend resorbiert werden kann. Das Strukturmittel hat vorzugsweise ein mittleres Molekulargewicht von höchstens 250.000, weiter bevorzugt höchstens 200.000 und am meisten bevorzugt höchstens 150.000. Ein mögliches Strukturmittel ist Vivapur oder genauer Vivapur 101. Polymerisierungsgrade können für diese Substanzen im Bereich von 180 bis 250 liegen und abhängig davon können Molekulargewichte variieren und im Bereich von ca. 50.000 liegen.

Das Strukturmittel ist vorzugsweise ausgewählt aus natürlichen oder synthetischen Polysacchariden, Polymethacrylaten und deren Copolymeren, Polyacrylaten und Mischungen daraus.

Besonders bevorzugt umfasst das Strukturmittel ein natürliches oder synthetisches Polysaccharid. Besonders bevorzugt besteht das Strukturmittel aus einem natürlichen oder synthetischen Polysaccharid. Bevorzugte Polysaccharide weisen mehr als 10 Monosaccharidbausteine auf. Als geeignete Monosaccharidbausteine haben sich Pentosen und Hexosen erwiesen, weiter bevorzugt ausgewählt aus Glucose, Galactose, Xylose, Fructose, Arabinose, Mannose, Mannuronsäure, Guluronsäure, Gulose und Mischungen daraus. Besonders bevorzugte natürliche oder synthetische Polysaccharide sind ausgewählt aus Cellulosen, Cellulosederivaten, Alginaten und Mischungen daraus. Ein besonders bevorzugtes Strukturmittel in dem magensaftlöslichen Anteil ist mikrokristalline Cellulose.

Der Gesamtanteil der Strukturmittel der beiden Anteile der Arzneiform soll vorzugsweise wenigstens 15 Gew.-%, weiter bevorzugt wenigstens 20 Gew.-%, noch weiter bevorzugt wenigstens 22 Gew.-% und besonders bevorzugt wenigstens 25 Gew.-% betragen. Wird eine zu geringe Menge des Strukturmittels eingesetzt, wird der gewünschte Effekt nicht erreicht. Bei zu großen Mengen wird der Wirkstoff nicht schnell genug freigesetzt und die Verarbeitbarkeit der Arzneiform kann erheblich erschwert sein. Daher soll der Anteil an Strukturmittel an der Arzneiform einen Wert von 55 Gew.-%, weiter bevorzugt 45 Gew.-% und besonders bevorzugt 35 Gew.-% nicht übersteigen.

Die Summe der Gewichtsanteile an Emulgator und Strukturmittel im formstabilen Anteil soll wenigstens 40 Gew.-% bezogen auf die Gesamtmasse des formstabilen Anteils betragen. Sowohl Emulgator als auch Strukturmittel tragen zur Modifikation der Freisetzung des Wirkstoffs bei. Daher ist es bevorzugt, dass die Summe der Anteile wenigstens 40 Gew.-% beträgt, um die Freisetzung des Wirkstoffs optimal zu modifizieren und Plasmaspitzenspiegel durch eine zu schnelle Freisetzung insbesondere bei längerer Verweilzeit der Arzneiform im Magen effektiv zu verhindern. Gerade das synergistische Zusammenwirken von Emulgator und Strukturmittel ermöglicht dabei eine optimale Freisetzung des Wirkstoffs bei optimaler Verarbeitbarkeit der Komponenten der Arzneiform. Wie oben beschrieben sind zu hohe Anteile an Emulgator ebenso wie zu hohe Anteile an Strukturmittel an der Arzneiform nachteilig für die Verarbeitbarkeit und die Freisetzung des Wirkstoffs. Weiter bevorzugt beträgt die Summe der Gewichtsanteile an Emulgator und Retardierungsmittel an der erfindungsgemäßen Arzneiform wenigstens 45%, noch weiter bevorzugt wenigstens 48% und ganz besonders bevorzugt wenigstens 52%.

Dabei hat sich ein Masseverhältnis von Emulgator zu Strukturmittel im formstabilen Anteil von wenigstens 1:10, bevorzugt wenigstens 1:5 und noch mehr bevorzugt wenigstens 1:2 sowie besonders bevorzugt von wenigstens 1:1,2 als vorteilhaft erwiesen. Das Masseverhältnis beträgt vorzugsweise höchstens 10:1, weiter bevorzugt höchstens 5:1, noch weiter bevorzugt höchstens 2:1 und besonders bevorzugt höchstens 1,2:1. In ganz besonders bevorzugten Ausführungsformen der erfindungsgemäßen Arzneiform beträgt das Masseverhältnis von Emulgator zu Strukturmittel im formstabilen Anteil etwa 1:1.

Das Masseverhältnis von Wirkstoff zu Strukturmittel im formstabilen Anteil beträgt erfindungsgemäß bevorzugt weniger als 1:1, weiter bevorzugt höchstens 1:1,2, noch weiter bevorzugt höchstens 1:1,5 und besonders bevorzugt höchstens 1:1,8. Bei zu geringen Anteilen an Strukturmittel im Verhältnis zum Wirkstoff wird die Freisetzung des Wirkstoffs nicht sehr stark modifiziert. Dann kann die Gefahr bestehen, dass zu viel Wirkstoff bereits im Magen freigesetzt wird mit der Folge toxischer Plasmaspiegel. Allerdings sollte das Masseverhältnis einen Wert von 1:20, weiter bevorzugt 1:15, noch weiter bevorzugt von 1:10 und besonders bevorzugt von 1:4 nicht unterschreiten. Bei zu hohen Mengenanteilen von Strukturmittel kann die Freisetzung des Wirkstoffs zu stark verzögert sein und dieser möglicherweise erst in tiefen Darmabschnitten freigesetzt werden. Dann werden nur unzureichende Plasmaspiegel erreicht. Außerdem wird die Arzneiform insgesamt zu groß und damit schwerer schluckbar.

### Bindemittel

### Bindemittel im formstabilen Anteil

Das Bindemittel ist in dem erfindungsgemäßen formstabilen Anteil vorzugsweise mit einem Gehalt von wenigstens 5 Gew.-%, weiter bevorzugt wenigstens 10 Gew.-% und besonders bevorzugt wenigstens 30 Gew.-% vertreten. Sein Gehalt beträgt vorzugsweise höchstens 50 Gew.-%, weiter bevorzugt höchstens 40 Gew.-% und besonders bevorzugt höchstens 35 Gew.-%.

Die erfindungsgemäß eingesetzten Bindemittel haben den Vorteil, dass sie sich sehr leicht mit den anderen Komponenten der erfindungsgemäßen Arzneiformen verarbeiten lassen und schon in geringen Mengen ein hervorragendes Ergebnis liefern. Außerdem erleichtern die Bindemittel die Verarbeitung der Komponenten der erfindungsgemäßen Arzneiformen und tragen dazu bei, die Arzneiformen insgesamt zu stabilisieren, vorzugsweise die mechanische Stabilität der Arzneiformen bei der Herstellung zu erhöhen. Die Bindemittel sind beispielsweise geeignet, den Schmelzpunkt einer Mischung von Wirkstoff und Emulgator während der Herstellung der erfindungsgemäßen Arzneiformen anzuheben, so dass eine weitere Bearbeitung dieser Mischung einfach möglich ist.

Geeignete Bindemittel können anorganischer oder organischer Art sein. Das Bindemittel ist vorzugsweise ein natürliches oder synthetisches Polysaccharid umfassend zwei oder mehrere gleiche oder verschiedene Monosaccharidbausteine, besonders bevorzugt ausgewählt aus Glucose, Galactose und Mischungen daraus. Solche Stoffe sind kostengünstig erhältlich, leicht verarbeitbar und zeigen teilweise gleichzeitig eine gewisse zerfallsfördernde Wirkung. Es ist besonders vorteilhaft, wenn das Bindemittel zugleich geeignet ist, einen schlechten Geschmack des Wirkstoffs zu maskieren. Bevorzugte Bindemittel haben daher einen süßen Geschmack.

Besonders bevorzugt ist das Bindemittel ausgewählt aus Calciumphosphat, Lactose, Stärke, Stärkederivaten und Mischungen daraus. Unter den Stärken sind Maisstärke und vorverkleisterte Stärke besonders bevorzugt wegen der einfachen Verarbeitbarkeit und vorteilhaften Eigenschaften. Ganz besonders bevorzugt ist das Bindemittel ausgewählt aus Stärke, Calciumphosphat und Mischungen daraus, weiter bevorzugt besteht das Bindemittel aus Stärke, Calciumphosphat oder Mischungen daraus.

Der Anteil dieses Bindemittels an der Arzneiform soll vorzugsweise wenigstens 10 Gew.-%, weiter bevorzugt wenigstens 15 Gew.-% und besonders bevorzugt wenigstens 20 Gew.-% betragen. Vorzugsweise soll der Anteil an Bindemittel an der Arzneiform einen Wert von 60 Gew.-%, weiter bevorzugt 55 Gew.-% und besonders bevorzugt 50 Gew.-% nicht übersteigen. Eine zu große Menge an Bindemittel vergrößert die Arzneiform und ist daher unerwünscht.

### Bindemittel im magensaftlöslichen Anteil

Auch der magensaftlösliche Anteil der Arzneiform soll vorzugsweise ein Bindemittel enthalten. Der Anteil des Bindemittels soll vorzugsweise wenigstens 30 Gew.-%, weiter bevorzugt wenigstens 40 Gew.-% und besonders bevorzugt wenigstens 50 Gew.-% betragen. Das Bindemittel fördert in der Regel auch den Zerfall der Arzneiform, so dass der Anteil in dem magensaftlöslichen Anteil vorzugsweise größer ist als in dem formstabilen Anteil. Formulierungsbedingt hat sich ergeben, dass ein maximaler Anteil von 70 Gew.-%, weiter bevorzugt 65 Gew.-% und besonders bevorzugt 60 Gew.-% an Bindemittel in dem magensaftlöslichen Anteil besonders vorteilhaft ist.

Die erfindungsgemäß eingesetzten Bindemittel haben den Vorteil, dass sie sich sehr leicht mit den anderen Komponenten der erfindungsgemäßen Arzneiformen verarbeiten lassen und schon in geringen Mengen ein hervorragendes Ergebnis liefern. Außerdem erleichtern die Bindemittel die Verarbeitung der Komponenten der erfindungsgemäßen Arzneiformen und tragen dazu bei, die Arzneiformen insgesamt zu stabilisieren, vorzugsweise die mechanische Stabilität der Arzneiformen bei der Herstellung zu erhöhen. Die Bindemittel sind beispielsweise geeignet, den Schmelzpunkt einer Mischung von Wirkstoff und Emulgator während der Herstellung der erfindungsgemäßen Arzneiformen anzuheben, so dass eine weitere Bearbeitung dieser Mischung einfach möglich ist.

Geeignete Bindemittel können anorganischer oder organischer Art sein. Das Bindemittel ist vorzugsweise ein natürliches oder synthetisches Polysaccharid umfassend zwei oder mehrere gleiche oder verschiedene Monosaccharidbausteine, besonders bevorzugt ausgewählt aus Glucose, Galactose und Mischungen daraus. Solche Stoffe sind kostengünstig erhältlich, leicht verarbeitbar und zeigen teilweise gleichzeitig eine gewisse zerfallsfördernde Wirkung. Es ist besonders vorteilhaft, wenn das Bindemittel zugleich geeignet ist, einen schlechten Geschmack des Wirkstoffs zu maskieren. Bevorzugte Bindemittel haben daher einen süßen Geschmack.

Besonders bevorzugt ist das Bindemittel ausgewählt aus Calciumphosphat, Lactose, Stärke, Stärkederivaten und Mischungen daraus. Unter den Stärken sind Maisstärke und vorverkleisterte Stärke besonders bevorzugt wegen der einfachen Verarbeitbarkeit und vorteilhaften Eigenschaften. Ganz besonders bevorzugt ist das Bindemittel ausgewählt aus Stärke, Calciumphosphat und Mischungen daraus, weiter bevorzugt besteht das Bindemittel aus Stärke, Calciumphosphat oder Mischungen daraus.

### Weiterer Wirkstoff

Die Arzneiform kann einen weiteren Wirkstoff enthalten, insbesondere sowohl in dem formstabilen als auch in dem magensaftlöslichen Anteil. Bei der Behandlung von Schwindel hat sich der Einsatz von Kombinationspräparaten bewährt. Der zweite Wirkstoff weist insbesondere eine vom ersten Wirkstoff deutlich abweichende Löslichkeit auf.

Grundsätzlich ist die Verarbeitung und Darreichung von Wirkstoffen mit voneinander abweichenden Löslichkeitseigenschaften problematisch. Es besteht beispielsweise die Gefahr, dass der besser lösliche Wirkstoff zu schnell aus der Arzneiform freigesetzt wird, während der schlechter lösliche Wirkstoff nicht oder nur unzureichend freigesetzt wird. Mit der erfindungsgemäßen Arzneiform ist es möglich, Wirkstoffe mit voneinander abweichenden Löslichkeitseigenschaften zu verarbeiten, selbst Wirkstoffe mit deutlich abweichenden Löslichkeitseigenschaften. Deutlich abweichende Löslichkeitseigenschaften liegen erfindungsgemäß vor, wenn der zweite Wirkstoff eine Löslichkeit hat, die bei pH 7 und 22°C in wässriger Lösung um wenigstens eine Zehnerpotenz höher liegt als die Löslichkeit des ersten Wirkstoffs bei pH 7 und 22°C in wässriger Lösung. Bevorzugt liegt die Löslichkeit des zweiten Wirkstoffs bei pH 7 und 22°C sogar um den Faktor 50 höher als die Löslichkeit des ersten Wirkstoffs, ganz besonders bevorzugt sogar um den Faktor 100, also zwei Zehnerpotenzen. Solche Wirkstoffe profitieren besonders von der erfindungsgemäßen Formulierung, weil ihre Verarbeitung zusammen mit gering löslichen Wirkstoffen in bisher bekannten Arzneiformen an Grenzen stößt.

Der zweite Wirkstoff ist bevorzugt mit einem Anteil von wenigstens 10 mg, weiter bevorzugt wenigstens 20 mg und besonders bevorzugt wenigstens 30 mg in dem formstabilen Anteil enthalten. Besonders bevorzugt ist er in einem Anteil von höchstens 100 mg, weiter bevorzugt höchstens 80 mg und besonders bevorzugt höchsten 60 mg in dem formstabilen Anteil enthalten. Damit ist der Anteil an zweitem Wirkstoff in dem formstabilen Anteil vorzugsweise größer als der Anteil des ersten Wirkstoffes in diesem Anteil. Vorzugsweise ist der Anteil des zweiten Wirkstoffs in dem formstabilen Anteil größer als der Anteil des zweiten Wirkstoffs in dem magensaftlöslichen Anteil.

Der Anteil des zweiten Wirkstoffs in dem magensaftlöslichen Anteil beträgt vorzugsweise wenigstens 10 mg, weiter bevorzugt wenigstens 15 mg und bevorzugt höchstens 40 mg, weiter bevorzugt höchstens 30 mg. Der Anteil des zweiten Wirkstoffs in dem magensaftlöslichen Anteil ist vorzugsweise größer als der Anteil des ersten Wirkstoffes in dem magensaftlöslichen Anteil der Arzneiform.

### Tensid/Triglycerid

In bevorzugten Ausführungsformen enthält die Arzneiform ferner wenigstens ein Tensid. Es hat sich gezeigt, dass bestimmte Tenside die Magenentleerung und damit die Freigabe des formstabilen Anteils in den Darm verlangsamen können, was die Wirkstoffabgabe weiter verlängert.

Bevorzugte Tenside sind ionische Tenside, insbesondere anionische Tenside. Metallseifen haben sich als besonders vorteilhaft erwiesen. Unter Metallseifen werden vorzugsweise Substanzen verstanden, die als anionische Komponente eine organische Säure und als kationische Komponente ein Metallkation umfassen. Das Metallkation ist vorzugsweise ausgewählt aus Alkali- und Erdalkalimetallionen. Besonders bevorzugt sind Alkalimetallionen und insbesondere Natrium und Kalium. Unter den Erdalkalimetallionen sind Magnesium und Calcium besonders bevorzugt.

Bevorzugte organische Säuren, die als anionische Komponente in den Tensiden vorkommen können, sind Säuren mit wenigstens 6 Kohlenstoffatomen. Organische Säuren mit kürzeren Kohlenstoffkettenlängen haben sich als weniger wirksam erwiesen. Die organischen Säuren sollen allerdings vorzugsweise Kettenlängen von 22 Kohlenstoffatomen nicht überschreiten. Bei längeren Kohlenstoffketten ist der synergistische Effekt im Zusammenspiel mit dem Emulgator nicht stark genug ausgeprägt. Besonders bevorzugt weisen die organischen Säuren in den Tensiden Kettenlängen von wenigstens 10 und höchstens 18, weiter bevorzugt wenigstens 12 und höchstens 16 Kohlenstoffatomen auf.

Die organischen Säuren können verzweigte oder unverzweigte Kohlenstoffketten aufweisen, bevorzugt sind die Kohlenstoffketten unverzweigt. Ein besonders bevorzugtes Tensid ist Natriummyristat.

Die erfindungsgemäßen Tenside haben die Aufgabe, die Magen-Darm-Passage des Speisebreis und damit die Resorption des ersten Wirkstoffes zu verlangsamen. Es wurde interessanterweise gefunden, dass dies besonders durch die hier beschriebenen Metallseifen erzielt werden kann. Daher sind die Tenside vorzugsweise Teil des magensaftlöslichen Anteils.

Der Anteil des Tensids an der erfindungsgemäßen Arzneiform soll vorzugsweise wenigstens 2 Gew.-%, bevorzugt wenigstens 7 Gew.-%, weiter bevorzugt wenigstens 12 Gew.-% und besonders bevorzugt wenigstens 17 Gew.-% betragen. Es hat sich gezeigt, dass die Effekte des Tensids so besonders ausgeprägt zutage treten. Eine Menge von vorzugsweise 35 Gew.-%, weiter bevorzugt 25 Gew.-% und besonders bevorzugt 20 Gew.-% sollte aber nicht überschritten werden. Dadurch würde die Freisetzung des Wirkstoffes zu stark behindert. Die absolute Masse des Tensids in der Arzneiform soll einen Wert von 300 mg, weiter bevorzugt 250 mg und besonders bevorzugt 200 mg nicht überschreiten. Die Mindestmenge des Tensids soll vorzugsweise einen Wert von 10 mg, weiter bevorzugt 50 mg und besonders bevorzugt 100 mg nicht unterschreiten.

Der Gehalt an Tensid sollte im Masseverhältnis zum Wirkstoff einen Anteil von 1 zu 1, weiter bevorzugt 1,5 zu 1 und besonders bevorzugt 2 zu 1 nicht unterschreiten. Bevorzugt soll dieses Verhältnis einen Wert von 5 zu 1, weiter bevorzugt 4 zu 1 und insbesondere 3 zu 1 nicht überschreiten. Werden diese Werte berücksichtigt, kann die oben skizzierte vorteilhafte Wirkung optimal genutzt werden.

In einer bevorzugten Ausführungsform weist die Arzneiform dieser Erfindung wenigstens ein Triglycerid auf. Das Triglycerid dient dazu, die Magen-Darm-Passage der Arzneiform zu verlangsamen. Das Triglycerid kann auch in Kombination mit dem Tensid eingesetzt werden, um die Freisetzung des Wirkstoffes weiter zu verlangsamen.

Das Triglycerid umfasst vorzugsweise einen Fettsäurerest mit wenigstens 10 Kohlenstoffatomen, weiter bevorzugt wenigstens 12 Kohlenstoffatomen und besonders bevorzugt wenigstens 16 Kohlenstoffatomen. Diese Mindestkettenlänge ist wünschenswert, weil damit die Verlängerung der Verweildauer der Arzneiform im Magen als Teil einer Arzneiform besonders ausgeprägt ist und die Freisetzung des Wirkstoffes so besonders effizient unterstützt werden kann. Allerdings sollte die Kettenlänge vorzugsweise einen Wert von 22 Kohlenstoffatomen, weiter bevorzugt 20 Kohlenstoffatomen nicht überschreiten. In besonders bevorzugten Ausführungsformen erfüllen alle Fettsäurereste in dem Triglycerid diese Voraussetzungen.

Das Triglycerid kann vorzugsweise in einem Anteil von wenigstens 10 Gew.-%, weiter bevorzugt wenigstens 15 Gew. % und besonders bevorzugt wenigstens 20 Gew.-% in der erfindungsgemäßen Arzneiform zum Einsatz kommen. Ein Höchstanteil von vorzugsweise 40 Gew.-%, weiter bevorzugt 30 Gew.-% und besonders bevorzugt 25 Gew.-% sollte aber nicht überschritten werden, weil andernfalls die Freisetzung des Wirkstoffes zu stark eingeschränkt würde.

Die absolute Masse des Triglycerids in der Arzneiform soll einen Wert von 500 mg, weiter bevorzugt 400 mg und besonders bevorzugt 300 mg nicht überschreiten. Die Mindestmenge des Triglycerids soll vorzugsweise einen Wert von 50 mg, weiter bevorzugt 100 mg und besonders bevorzugt 150 mg nicht unterschreiten.

Der Gehalt an Triglycerid sollte im Masseverhältnis zum Wirkstoff einen Anteil von 2 zu 1, weiter bevorzugt 3 zu 1 und besonders bevorzugt 3,5 zu 1 nicht unterschreiten. Bevorzugt soll dieses Verhältnis einen Wert von 20 zu 1, weiter bevorzugt 10 zu 1 und insbesondere 7 zu 1 nicht überschreiten. Werden diese Werte berücksichtigt, kann die oben skizzierte vorteilhafte Wirkung optimal genutzt werden.

### Beispiele

### Beispiel 1 - Schichttablette

| **Schicht 1** | **formstabiler Anteil** | |
|---|---|---|
| **Gehalt** | **Komponente** | **Funktion** |
| 40,0 mg | Dimenhydrinat | Zweiter Wirkstoff |
| 80,0 mg | Methylcellulose | Strukturmittel |
| 40,0 mg | Maisstärke | Bindemittel |
| 80,0 mg | Gelucire 50/13 | Emulgator |
| 20,0 mg | Cinnarizin | Wirkstoff |
| 2,6 mg | Aerosil | Fließmittel |
| 5,2 mg | Mg-Stearat | Gleitmittel |
| 10,4 mg | Talkum | Formtrennmittel |
| 278,2 mg | | |

| **Schicht 2** | **Magensaftlöslicher Anteil** | |
|---|---|---|
| **Gehalt** | **Komponente** | **Funktion** |
| 20,0 mg | Dimenhydrinat | Zweiter Wirkstoff |
| 40,0 mg | Mikrokristalline Cellulose | Strukturmittel |
| 25,0 mg | Vorverkleisterte Stärke | Bindemittel |
| 25,2 mg | Calciumphosphat | Bindemittel |
| 10,0 mg | Cinnarizin | Wirkstoff |
| 0,6 mg | Aerosil | Fließmittel |
| 1,0 mg | Mg-Stearat | Gleitmittel |
| 121,8 mg | | |

Zur Herstellung einer erfindungsgemäßen Schichttablette der oben genannten Zusammensetzung wird zunächst der formstabile Anteil hergestellt. Zu diesem Zweck wird der zweite Wirkstoff, das Strukturmittel und das Bindemittel gemischt. Diese Mischung wird zusammen mit dem Emulator und dem Wirkstoff auf 75 °C erhitzt, um eine Schmelzgranulation durchzuführen. Nach der Schmelzgranulation wird das Granulat abgekühlt und gesiebt. Dieses gesiebte Granulat (bevorzugte Korngröße 1,0 mm) wird mit einer ebenfalls zuvor gesiebten Mischung aus Fließmittel, Gleitmittel und Formtrennmittel gemischt. Die so erhaltene Zusammensetzung wird zusammen mit den Komponenten des magensaftlöslichen Anteils verpresst. Die Komponenten des magensaftlöslichen Anteils werden zu diesem Zweck gemischt und anschließend gesiebt. Hier offenbart sich ein Vorteil dergestalt, dass der magensaftlösliche Anteil einfach herstellbar ist. Die so hergestellte Arzneiform zeichnete sich durch außerordentliche lange Wirkdauer bei Einnahme zu oder nach einer Mahlzeit aus.

### Beispiel 2 - Mantel-Kern-Tablette

| **Kern** | **formstabiler Anteil** | |
|---|---|---|
| **Gehalt** | **Komponente** | **Funktion** |
| 20,0 mg | Dimenhydrinat | Zweiter Wirkstoff |
| 41,3 mg | Mikrokristalline Cellulose | Strukturmittel |
| 60,0 mg | Maisstärke | Bindemittel |
| 40,0 mg | Gelucire 50/13 | Emulgator |
| 10,0 mg | Cinnarizin | Wirkstoff |
| 1,2 mg | Aerosil | Fließmittel |
| 2,5 mg | Mg-Stearat | Gleitmittel |
| 5,0 mg | Talkum | Formtrennmittel |
| 180,0 mg | | |

| **Mantel** | **Magensaftlöslicher Anteil** | |
|---|---|---|
| **Gehalt** | **Komponente** | **Funktion** |
| 40,0 mg | Dimenhydrinat | Zweiter Wirkstoff |
| 150,0 mg | Mikrokristalline Cellulose | Strukturmittel |
| 100,0 mg | Vorverkleisterte Stärke | Bindemittel |
| 177,5 mg | Calciumphosphat | Bindemittel |
| 20,0 mg | Cinnarizin | Wirkstoff |
| 2,5 mg | Aerosil | Fließmittel |
| 5,0 mg | Mg-Stearat | Gleitmittel |
| 5,0 mg | Talkum | Formtrennmittel |
| 500,0 mg | | |

Die Herstellung einer erfindungsgemäßen Mantel-Kern-Tablette gemäß der beschriebenen Zusammensetzung erfolgte durch Mischen der Bestandteile zweiter Wirkstoff, Strukturmittel und Bindemittel. Die Mischung wurde sodann auf 75 °C erhitzt und mit einer Mischung von Wirkstoff und Emulator granuliert. Dem schloss sich ein Schritt des Abkühlens und Siebens an. Das gesiebte Granulat wurde mit einer ebenfalls gesiebten Mischung von Fließmittel, Gleitmittel und Formtrennmittel gemischt und zu Tabletten geformt. Auf diese Tabletten wurde ein Film aufgebracht, der ein Acrylatpolymer, einen Weichmacher und Talkum enthielt. Die so erhaltenen Tabletten stellen den Kern und damit den formstabilen Anteil dieser Arzneiform dar. Zur Herstellung des magensaftlöslichen Anteils, hier also des Mantels, wurden alle Bestandteile des Mantels gemischt und um den beschichteten Kern gepresst.

### Figurenbeschreibung

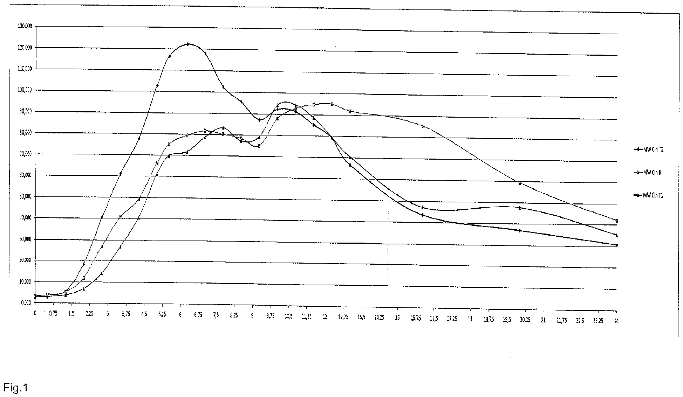

Figur 1 zeigt den Plasmaspiegelverlauf (in vivo) des Wirkstoffes Cinnarizin nach Einnahme einer herkömmlichen Arzneiform (Kurve MW Cin R), wobei nach 0 Stunden, 5 Stunden und 10 Stunden der Wirkstoff verabreicht wurde. Die beiden anderen Kurven MW Cin T1 und MW Cin T2 beschreiben den Plasmaspiegelverlauf für erfindungsgemäße Arzneiformen. Es ist sehr schön zu erkennen, dass die erfindungsgemäßen Arzneiformen eine Freisetzung über einen sehr langen Zeitraum ermöglichen.

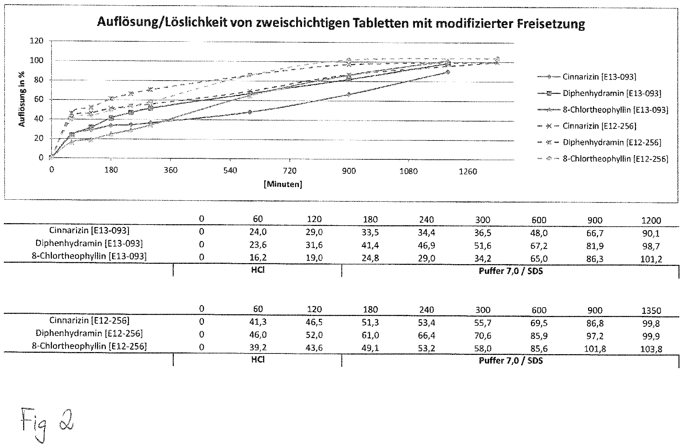

Figur 2 zeigt in vitro Daten für zwei erfindungsgemäße Arzneiformen. Es geht um die Auflösung, d.h. das Löslichkeitsverhalten für zwei verschiedene Arzneiformen und nicht nur für den Wirkstoff Cinnarizin sondern auch für Diphenhydramin und Theophyllin. Es handelt sich um zwei Ausführungsformen, wobei E12-256 eine bevorzugte Ausführungsform ist.

Die Kurve MW Cin T2 korreliert zu Cinnarizin-Anteil der Arzneiform E12-256, während die Kurve MW Cin T1 zu dem Cinnarizin-Anteil der Arzneiform E13-093 korreliert.

## Patentansprüche

1. Arzneiform umfassend
• einen Wirkstoff oder ein Salz davon,
• einen formstabilen Anteil, der sich in einem wässrigen Medium bei einem pH-Wert von <5 während einer Dauer von 2 Stunden auch unter Rühren nicht auflöst und unter diesen Bedingungen nicht in einem Maße erodiert, dass sein kleinster Durchmesser auf einen Wert von <5 mm sinkt,
• einen magensaftlöslichen Anteil, der sich in einem wässrigen Medium bei einem pH-Wert von <5 während einer Dauer von weniger als 2 Stunden auflöst oder unter diesen Bedingungen in einem Maße erodiert, dass sein größter Durchmesser auf einen Wert von <2 mm sinkt,
wobei sowohl der formstabile als auch der magensaftlösliche Anteil eine Teilmenge des Wirkstoffes enthalten,
zur Anwendung in einem Verfahren zur Behandlung von Schwindel beim Menschen durch höchstens zweimal tägliche Verabreichung an einen Patienten zu oder nach einer Mahlzeit.

2. Arzneiform nach Anspruch 1, wobei die Verabreichung einmal täglich erfolgt.

3. Arzneiform nach Anspruch 1 oder 2, wobei der Patient älter als 40 Jahre ist.

4. Arzneiform nach einem der Ansprüche 1 bis 3, wobei der Patient älter als 50 Jahre ist.

5. Arzneiform nach wenigstens einem der vorhergehenden Ansprüche, wobei der Wirkstoff ausgewählt ist aus Wirkstoffen der Wirkstoffklassen Antihistaminika, Antiemetika, Antivertiginosa und/oder Calciumkanalblocker

6. Arzneiform nach einem der vorhergehenden Ansprüche, wobei der Wirkstoff Cinnarizin ist, insbesondere in einer Menge von wenigstens 40 mg bezogen auf die Cinnarizin-Base.

7. Arzneiform nach einem der vorhergehenden Ansprüche, ausgestaltet als Schichttablette, wobei der formstabile und der magensaftlösliche Anteil je eine Schicht darstellen, oder ausgestaltet als Mantel-Kern-Tablette, wobei der formstabile Anteil den Kern und der magensaftlösliche Anteil den Mantel darstellt.

8. Arzneiform nach wenigstens einem der vorhergehenden Ansprüche, wobei die Verabreichung in einem Zeitfenster von bis zu 1 Stunde nach Einnahme einer Mahlzeit erfolgt.

9. Arzneiform nach wenigstens einem der vorhergehenden Ansprüche, wobei sowohl in dem formstabilen Anteil als auch in dem magensaftlöslichen Anteil ein weiterer Wirkstoff enthalten ist.

10. Arzneiform nach wenigstens einem der vorhergehenden Ansprüche, wobei die Mahlzeit hinsichtlich ihrer Zusammensetzung folgende Eigenschaften aufweist:
| | |
|---|---|
| a. Kohlenhydrate: | >50 g |
| b. Fett: | >10 g |
| c. Protein: | >10 g |

11. Arzneiform einem der vorhergehenden Anteil, wobei der Anteil des Wirkstoffes in dem magensaftlöslichen Anteil etwa 20% bis 65% der Wirkstoffmenge in dem formstabilen Anteil entspricht.

12. Arzneiform nach wenigstens einem der vorhergehenden Ansprüche, wobei das wässrige Medium Phosphatpuffer pH 4,5 R des Europäischen Arzneibuchs 7.0 ist.

13. Arzneiform nach wenigstens einem der vorhergehenden Ansprüche, das frei von Antioxidantien und/oder Komplexbildnern ist.

## Claims

1. A medicament form, comprising
• an active ingredient or a salt thereof,
• a dimensionally stable portion which does not dissolve in an aqueous medium at a pH value of < 5 during a duration of 2 hours also under stirring and under these conditions does not erode in an extent that its smallest diameter decreases to a value of < 5 mm,
• a gastric juice-soluble portion which dissolves in an aqueous medium at a pH value of < 5 during a duration of shorter than 2 hours or under these conditions erodes in an extent that its largest diameter decreases to a value of < 2 mm,
wherein both, the dimensionally stable and also the gastric juice-soluble portions contain a partial amount of the active ingredient, for use in a method for treating vertigo in a human being by an administration to a patient at most two times a day during or after a meal.

2. The medicament form according to claim 1, wherein the administration is conducted once a day.

3. The medicament form according to claim 1 or 2, wherein the patient is older than 40 years.

4. The medicament form according to one of claims 1 to 3, wherein the patient is older than 50 years.

5. The medicament form according to at least one of the preceding claims, wherein the active ingredient is selected from active ingredients of the active ingredient classes of antihistamins, antiemetics, antivertiginous drugs and/or calcium channel blockers.

6. The medicament form according to one of the preceding claims, wherein the active ingredient is cinnarizine, in particularly in an amount of at least 40 mg, based on the cinnarizine base.

7. The medicament form according to one of the preceding claims, designed as layer tablet, wherein the dimensionally stable and the gastric juice-soluble portions each represent one layer, or designed as shell-core tablet, wherein the dimensionally stable portion represents the core and the gastric juice-soluble portion represents the shell.

8. The medicament form according to at least one of the preceding claims, wherein the administration is conducted in a time frame of up to 1 hour after the ingestion of a meal.

9. The medicament form according to at least one of the preceding claims, wherein in both, the dimensionally stable portion and also the gastric juice-soluble portion a further active ingredient is contained.

10. The medicament form according to at least one of the preceding claims, wherein the meal with respect to its composition has the following properties:
| | |
|---|---|
| a. carbohydrates: | > 50 g |
| b. fat: | > 10 g |
| c. protein: | > 10 g. |

11. The medicament form with the preceding portion, wherein the portion of the active ingredient in the gastric juice-soluble portion corresponds to about 20 % to 65 % of the amount of active ingredient in the dimensionally stable portion.

12. The medicament form according to at least one of the preceding claims, wherein the aqueous medium is phosphate buffer pH 4.5 R of the European Pharmacopoeia 7.0.

13. The medicament form according to at least one of the preceding claims, which is free of antioxidants and/or complexing agents.

## Revendications

1. Forme médicamenteuse comprenant
• un principe actif ou un sel de celui-ci,
• une fraction de forme stable qui ne se dissout pas dans un milieu aqueux à un pH inférieur à 5 pendant une durée de 2 heures même sous agitation et qui ne s'érode pas dans ces conditions à un degré tel que son plus petit diamètre ait une valeur inférieure à 5 mm,
• une fraction, soluble dans le suc gastrique, qui se dissout dans un milieu aqueux à un pH inférieur à 5 pendant une durée inférieure à 2 heures ou qui s'érode dans ces conditions à un degré tel que son plus grand diamètre diminue jusqu'à une valeur inférieure à 2 mm,
la fraction soluble dans le suc gastrique et la fraction de forme stable contenant une quantité partielle du principe actif,
la forme médicamenteuse étant destinée à être utilisée dans un procédé de traitement des étourdissements chez l'être humain par administration à un patient à raison de deux fois par jour maximum pendant ou après un repas.

2. Forme médicamenteuse selon la revendication 1, l'administration se faisant une fois par jour.

3. Forme médicamenteuse selon la revendication 1 ou 2, le patient ayant plus de 40 ans.

4. Forme médicamenteuse selon l'une des revendications 1 à 3, le patient ayant plus de 50 ans.

5. Forme médicamenteuse selon l'une au moins des revendications précédentes, le principe actif étant choisi parmi les principes actifs des classes de principes actifs des antihistaminiques, des antiémétiques, des anti-vertigineux osa et/ou des inhibiteurs calciques.

6. Forme médicamenteuse selon l'une des revendications précédentes, le principe actif étant la cinnarizine, en particulier dans une quantité d'au moins 40 mg sur la base de la cinnarizine.

7. Forme médicamenteuse selon l'une des revendications précédentes, conçue sous forme de comprimés en couches, la fraction de forme stable et la fraction soluble dans le suc gastrique représentant chacune une couche, ou réalisée sous la forme d'un comprimé à noyau et enveloppe, la fraction de forme stable étant le noyau et la fraction soluble dans le suc gastrique étant l'enveloppe.

8. Forme médicamenteuse selon l'une au moins des revendications précédentes, l'administration se faisant dans une fenêtre de temps allant jusqu'à 1 heure après la prise d'un repas.

9. Forme médicamenteuse selon l'une au moins des revendications précédentes, un autre principe actif étant contenu à la fois dans la fraction de forme stable et dans la fraction soluble dans le suc gastrique.

10. Forme médicamenteuse selon l'une au moins des revendications précédentes, le repas présentant les propriétés suivantes en ce qui concerne sa composition :
a. Glucides : plus 50 g
b. Graisse : moins de 10 g
c. Protéines : plus de 10 g

11. Forme médicamenteuse selon l'une des revendications précédentes, la proportion de principe actif dans la fraction soluble dans le suc gastrique correspondant à un pourcentage d'environ 20 % à 65% de la quantité de principe actif dans la fraction de forme stable.

12. Forme médicamenteuse selon l'une au moins des revendications précédentes, le milieu aqueux étant un tampon phosphate de pH 4,5 R de la pharmacopée européenne 7.0.

13. Forme médicamenteuse selon l'une au moins des revendications précédentes, laquelle est exempte d'antioxydants et/ou d'agents complexants.
